(19) Europäisches Patentamt
European Patent Office
Office européen des brevets

(11) Numéro de publication: **0 543 713 B1**

(12) # FASCICULE DE BREVET EUROPEEN

(45) Date de publication de fascicule du brevet: **22.02.95**

(51) Int. Cl.[6]: **C07C 233/51**, A61K 31/165

(21) Numéro de dépôt: **92403073.7**

(22) Date de dépôt: **16.11.92**

(54) **Nouveaux dérivés de benzoate d'éthanolamine, leur procédé de préparation et les compositions pharmaceutiques les renfermant.**

(30) Priorité: **22.11.91 FR 9114357**

(43) Date de publication de la demande:
**26.05.93 Bulletin 93/21**

(45) Mention de la délivrance du brevet:
**22.02.95 Bulletin 95/08**

(84) Etats contractants désignés:
**AT BE CH DE DK ES FR GB GR IE IT LI LU NL PT SE**

(56) Documents cités:
**DE-A- 2 629 752**
**FR-A- 1 517 587**
**US-A- 4 237 165**

(73) Titulaire: **ADIR ET COMPAGNIE**
**1 rue Carle Hébert**
**F-92415 Courbevoie Cédex (FR)**

(72) Inventeur: **Wierzbicki, Michel**
**8 Résidence du Chemin Pavé**
**F-78620 l'Etang La Ville (FR)**
Inventeur: **Hugon, Pierre**
**13 rue Eugène Sue**
**F-92500 Rueil Malmaison (FR)**
Inventeur: **Duhault, Jacques**
**14bis Paul Demange**
**F-78290 Croissy Sur Seine (FR)**
Inventeur: **Boulanger, Michelle**
**10 Place du Général de Gaulle**
**F-78400 Chatou (FR)**
Inventeur: **Lacour, Françoise**
**44 avenue du Château**
**F-94300 Vincennes (FR)**

(74) Mandataire: **Reverbori, Marcelle**
**ADIR**
**1, rue Carle Hébert**
**F-92415 Courbevoie Cédex (FR)**

EP 0 543 713 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention a pour objet de nouveaux dérivés de benzoate d'éthanolamine, leur procédé de préparation et les compositions pharmaceutiques les renfermant.

Elles concernent particulièrement les dérivés de benzoate d'éthanolamine de formule générale I :

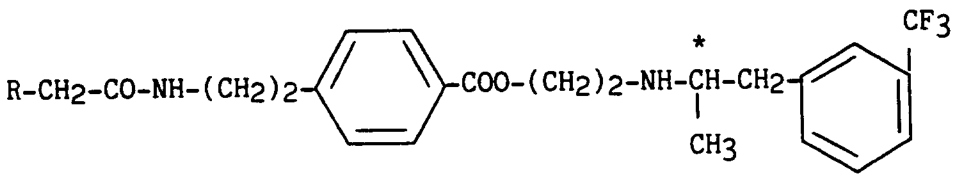

(I)

dans laquelle R représente :

a) soit un radical de formule :

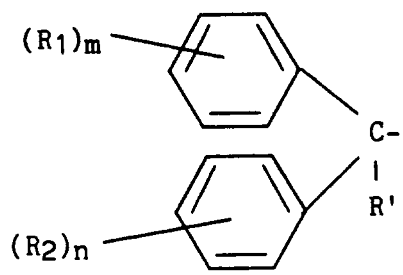

dans laquelle :

- R' represente un atome d'hydrogène ou un radical alkyle contenant de 1 à 5 atomes de carbone en chaine droite ou ramifiée,
- $R_1$ et $R_2$, identiques ou différents, représentent chacun un atome d'hydrogène ou un radical alkyle ou alkoxy ayant chacun de 1 à 5 atomes de carbone en chaine droite ou ramifiée, et
- m et n, identiques ou différents, représentent chacun 1, 2 ou 3 ;

b) soit un radical fluorényle de formule :

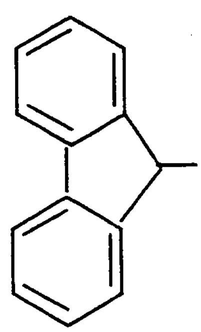

sous forme de composé racémique ou d'énantiomères.

L'état antérieur de la technique est illustré notamment :

- par les brevets français 1.517.587 et 6564 M qui ont respectivement pour objet :

. les composés de formule A :

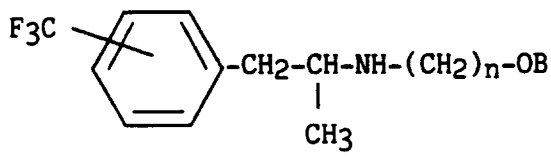

(A)

dans laquelle :

- n prend, entre autres, la valeur 2, et
- B représente un atome d'hydrogène ou un groupe COB', B' étant entre autres, un radical phényle éventuellement substitué, et

. l'utilisation des composés A à titre de médicaments dans le traitement de l'obésité, de la douleur et de l'épilepsie ; et

- par le brevet américain 4.237.165 qui concerne les compositions pharmaceutiques renfermant soit le 1-(3-trifluorométhylphényl)-2-($\beta$-hydroxyéthyl) aminopropane soit le 1-(3-trifluorométhylphényl)-2-($\beta$-benzoyloxyéthyl)aminopropane, utilisables dans le traitement des troubles du métabolisme.

Des modifications importantes de structure ont conduit aux dérivés de formule I de la présente invention, lesquels régularisent le métabolisme des glucides et des lipides et s'opposent à l'oxydation des LDL (lipoprotéines de faible densité) ce qui n'est pas le cas des composés de l'état de la technique ci-dessus cités qui sont eux inactifs sur l'oxydation des LDL.

La présente invention a également pour objet le procédé de préparation des composés de formule générale I caractérisé en ce que :

- l'on transforme l'acide de formule générale II :

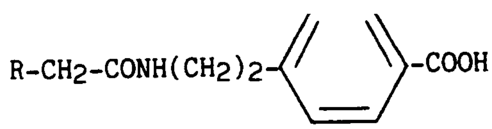

(II)

dans laquelle R a la signification précédemment définie, en un sel de formule générale II' :

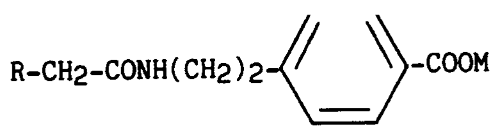

(II')

dans laquelle R a la signification précédemment définie et M est un métal alcalin ou alcalino-terreux ;

- l'on fait réagir ce dernier avec un composé halogéné de formule générale III :

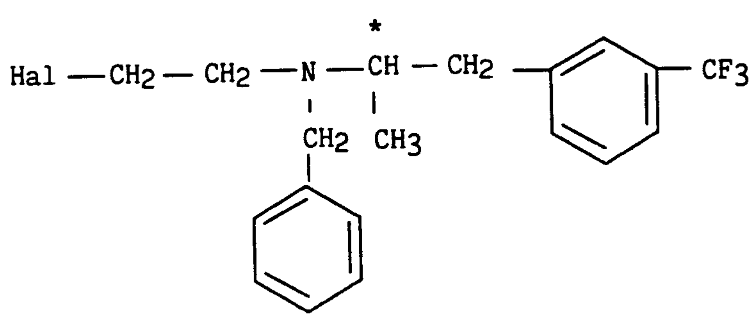

(III)

dans laquelle Hal représente un atome d'halogène tel qu'un atome de chlore, de brome ou d'iode ;

- et l'on débenzyle catalytiquement le composé ainsi obtenu de formule générale IV :

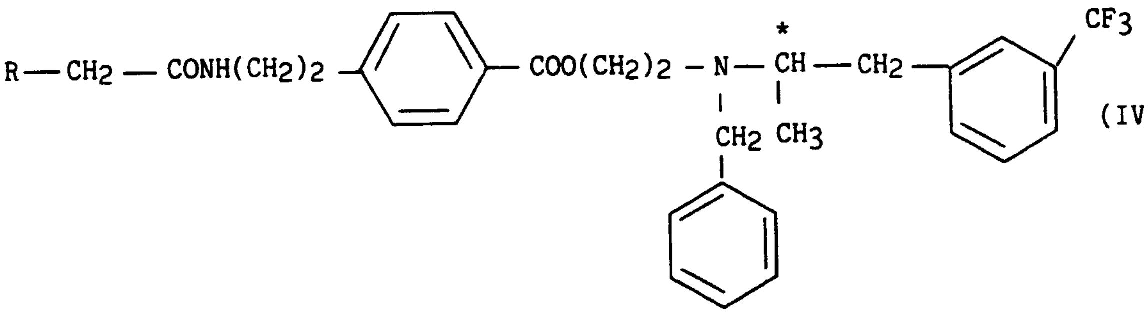

(IV)

dans laquelle R a la signification précédemment définie.

Certaines matières premières de formule générale II sont connues. C'est le cas des acides de formule générale II dans laquelle R représente :

- soit le radical benzhydryle, cf : European Journal of Pharmacology (1987), 141-2, 243-251 ;
- soit le radical fluorén-9-yle, cf : brevet US 4.136.197.

Les matières premières de formule générale II dans laquelle R représente un groupe benzhydryle substitué de formule :

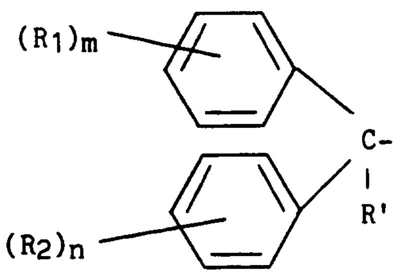

dans laquelle :

- m et n ont les significations précédemment définies et
- $R_1$, $R_2$ et R' sont tels que précédemment définis mais, de plus, ne représentent jamais simultanément hydrogène, c'est à dire les matières premières répondant plus précisément à la formule générale IIa :

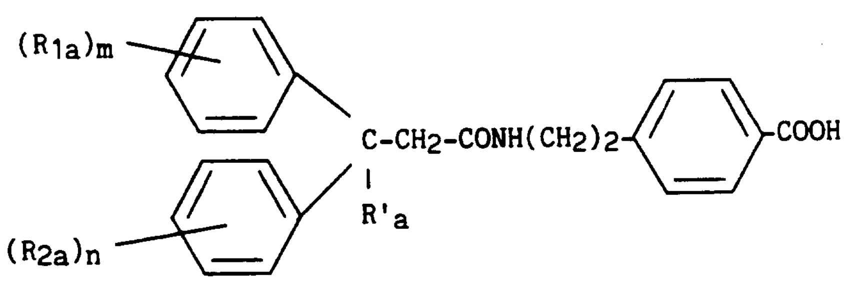

(IIa)

dans laquelle :

- m et n ont les significations précédemment définies,
- $R'_a$ représente un atome d'hydrogène ou un radical alkyle contenant de 1 à 5 atomes de carbone en chaine droite ou ramifiée,
- $R_{1a}$ et $R_{2a}$, identiques ou différents représentent chacun un atome d'hydrogène ou un radical alkyle ou alkoxy ayant chacun de 1 à 5 atomes de carbone en chaine droite ou ramifiée, à condition toutefois que $R_{1a}$ , $R_{2a}$ et $R'_a$ ne représentent jamais simultanément un atome d'hydrogène,

ont été préparées en transformant les acides de formule générale V :

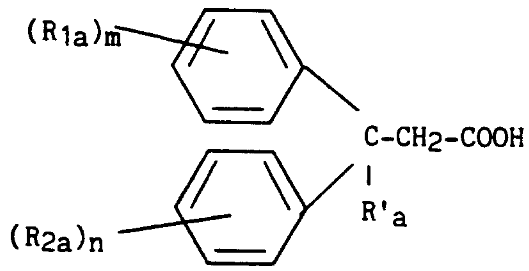

(V)

dans laquelle : $R'_a$, $R_{1a}$, $R_{2a}$, m et n ont les significations précédemment définies, en chlorure d'acide ou en anhydride mixte, respectivement de formule générale Va ou Vb :

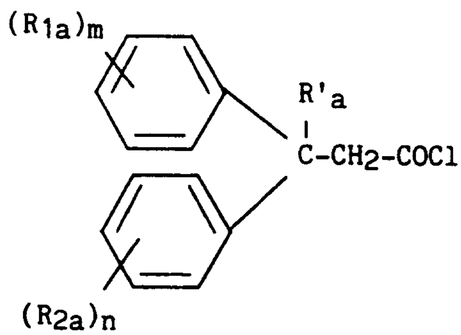

(Va)

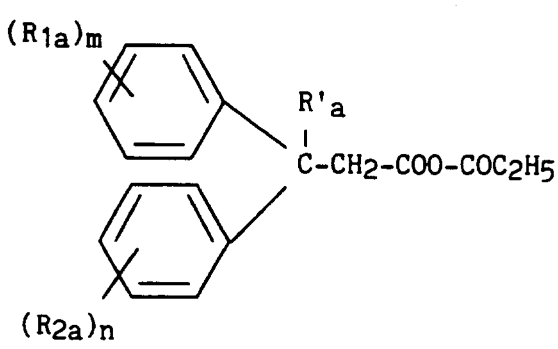

(Vb)

lesquels sont condensés

a) soit sur un amino acide de formule VIa :

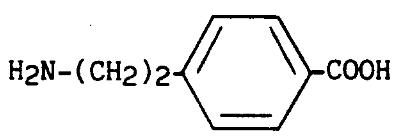

(VIa)

pour obtenir directement un composé de formule IIa, formule que l'on peut écrire plus précisément comme suit :

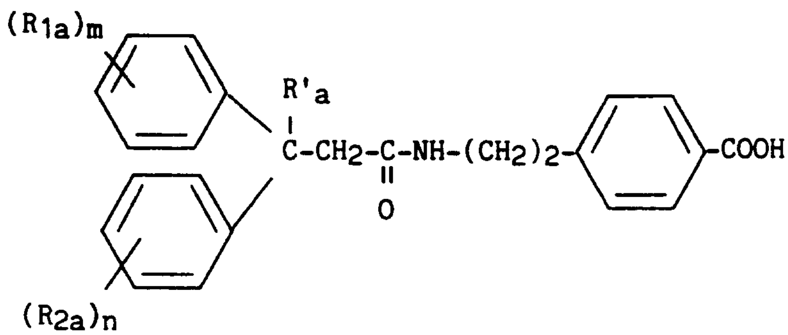

(IIa)

dans laquelle R'a, $R_{1a}$, $R_{2a}$, m et n ont les significations précédemment définies.

b) soit sur un amino ester de formule VIb :

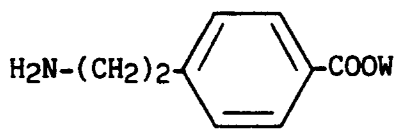

(VIb)

dans laquelle W est un radical alkyle ayant de 1 à 5 atomes de carbone en chaine droite ou ramifiée pour obtenir un composé de formule générale II'a :

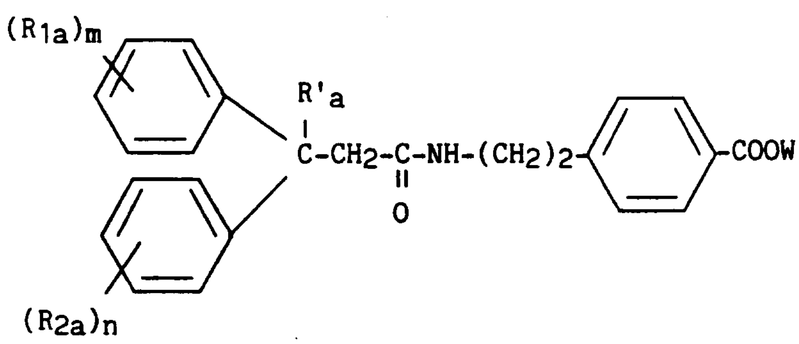

(II'a)

dans laquelle R'a, $R_{1a}$, $R_{2a}$, m, n et W ont les significations précédemment définies, lequel est hydrolysé en acide de formule IIa.

Dans le cas ou R'a représente un atome d'hydrogène, les acides V correspondants ont eux-mêmes été obtenus par réaction de HORNER entre la cétone de formule B :

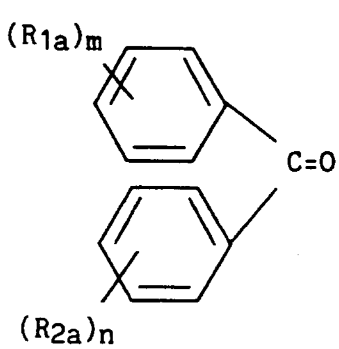

(B)

et le phosphonoacétate d'éthyle [$(C_2H_5O)_2OP-CH_2-COOC_2H_5$] réaction suivie d'une hydrogénation catalytique du composé ainsi formé de formule C :

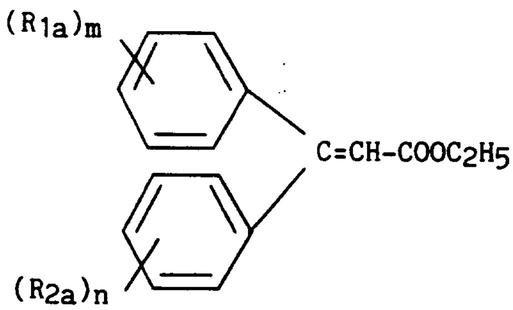

(C)

pour donner l'ester de formule D :

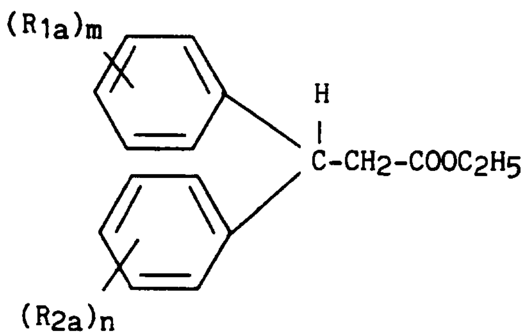

(D)

lequel est ensuite hydrolysé en acide correspondant de formule E :

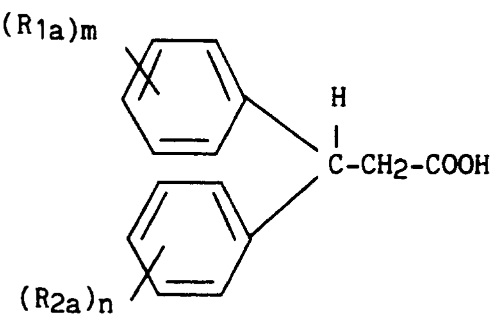

(E)

($R_{1a}$, $R_{2a}$, m et n ayant, dans toutes ces formules, les définitions précédemment définies), c'est à dire en acide V dans le cas où $R'_a$ représente un atome d'hydrogène.

Dans le cas où $R'_a$ représente un radical alkyle contenant de 1 à 5 atomes de carbone en chaine droite ou ramifiée, les acides V correspondants ont eux-mêmes été obtenus par condensation de COPE entre la cétone de formule F :

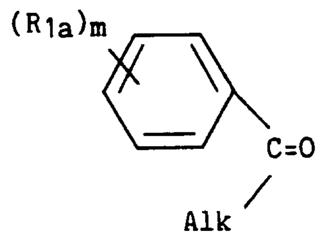

(F)

(dans laquelle $R_{1a}$ et m sont tels que précédemment définis et Alk représente un radical alkyle contenant de 1 à 5 atomes de carbone en chaine droite ou ramifiée)
et le cyanoacétate d'éthyle ($NC\text{-}CH_2\text{-}COOC_2H_5$) dans le toluène, en présence d'acide acétique et d'acétate d'ammonium pour donner le dérivé éthylénique de formule G :

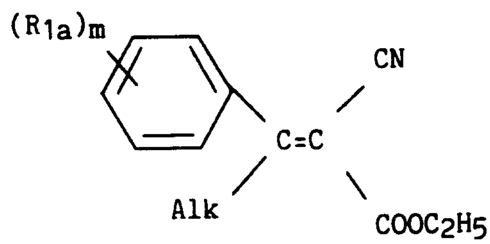

(G)

lequel est alors soumis à l'action d'un composé organomagnésien de formule H :

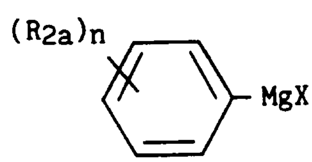

(H)

($R_{2a}$ et n étant tels que précédemment définis et X étant un atome d'halogène)
pour donner le composé de formule J :

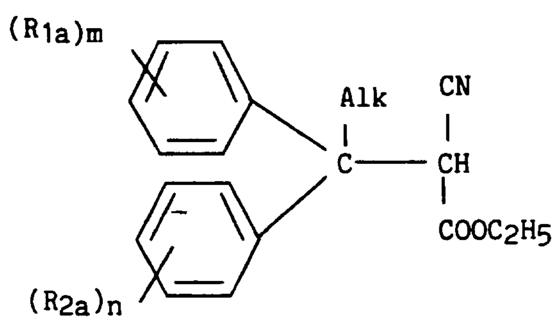

(J)

(dans laquelle $R_{1a}$, $R_{2a}$, m, n et Alk sont tels que précédemment définis) lequel composé J est hydrolysé en acide de formule K :

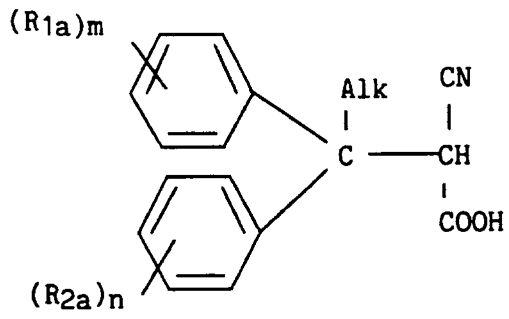

(K)

qui est décarboxylé pour donner le nitrile de formule L :

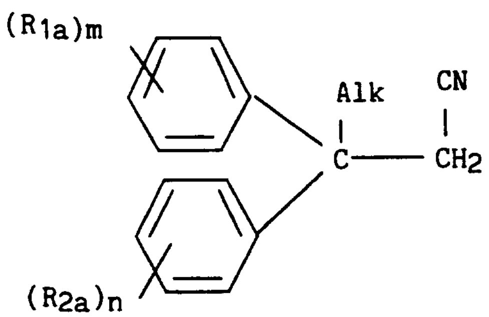

(L)

lequel est à son tour hydrolysé en acide attendu de formule M :

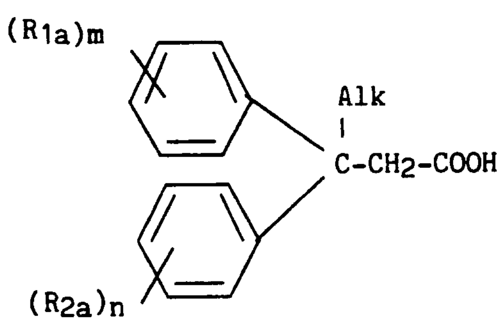

(M)

(dans laquelle $R_{1a}$, $R_{2a}$, m, n et Alk sont tels que précédemment définis) ; c'est à dire en acide de formule V dans le cas où $R'_a$ représente un radical alkyle contenant de 1 à 5 atomes de carbone en chaine droite ou ramifiée.

Les matières premières de formule générale III :

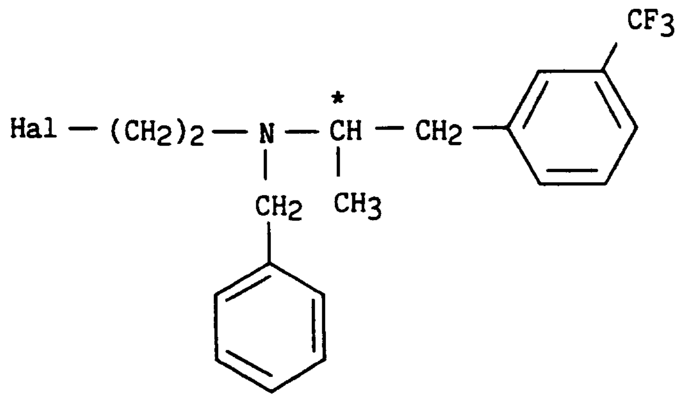

(III)

dans laquelle Hal a la signification précédemment définie, ont été préparées :

- en faisant réagir le (RS), (R) ou (S) 1-m-trifluorométhylphényl-2-benzylamino propane de formule N :

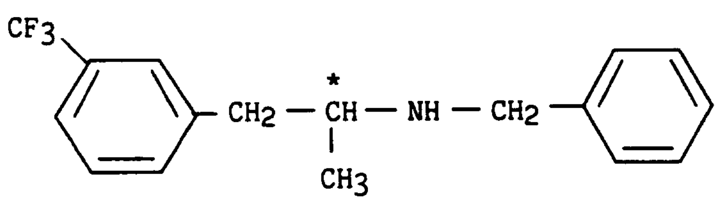

(N)

- avec un dérivé halogéné de formule générale P :

  $Hal\text{-}CH_2\text{-}COOAlk$ (P)

  dans laquelle Hal a la signification précédemment définie et Alk représente un radical alkyle ayant de 1 à 3 atomes de carbone,
- pour obtenir le glycinate de formule générale Q :

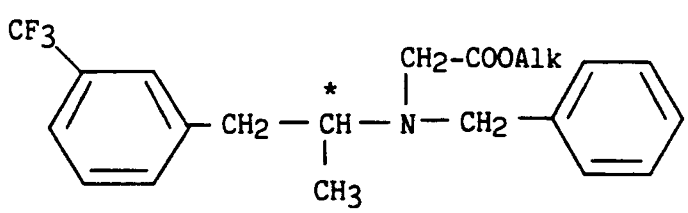

(Q)

- qui peut être transformé en glycine correspondante de formule T:

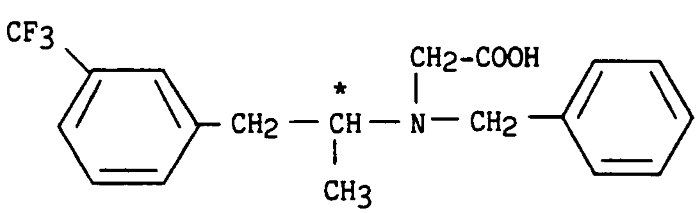

(T)

- lesquels composés de formule Q et T, soumis à réduction au moyen de LiAlH4 dans l'éther, donnent les composés (RS), (R) ou (S) de formule U :

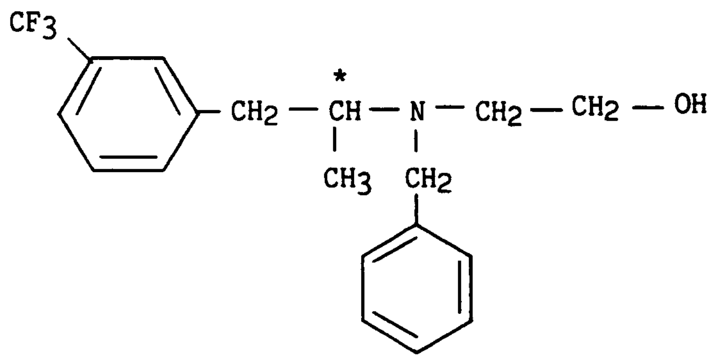

(U)

- Ce dernier est ensuite transformé en halogénure III, au moyen d'un composé halogéné tel que par exemple $SOCl_2$, $PCl_5$ ou $POCl_3$.

Les (S) et (R) 1-m-trifluorométhylphényl 2-benzylaminopropanes de formule (N) ont été préparés à partir du composé racémique correspondant ; le dédoublement étant réalisé selon des méthodes classiques au moyen par exemple d'acide d(+) camphosulfonique et d'acide d(-) dibenzoyltartrique.

Les composés de formule générale I peuvent être transformés en sels d'addition avec les acides, sels qui font, à ce titre, partie de la présente invention.

Comme acides utilisables pour la formation de ces sels, on peut citer, par exemple, dans la série minérale, les acides chlorhydrique, bromhydrique, sulfurique, nitrique, phosphorique et dans la série organique, les acides acétique, propionique, maléique, fumarique, tartrique, oxalique, benzoïque, méthane sulfonique et iséthionique.

Les composés de formule générale I et leurs sels d'addition physiologiquement tolérables possèdent des propriétés pharmacologiques et thérapeutiques intéressantes, notamment des propriétés régulatrices du métabolisme des glucides et des lipides. De plus, ils provoquent une diminution modérée de la pression artérielle.

Plus précisément, les composés de la présente invention améliorent l'efficacité de l'insuline à un niveau périphérique et/ou hépatique, d'où une amélioration de la tolérance au glucose et de l'hyperglycémie modérée, lorsqu'elle existe, sans risque d'hypoglycémie, ainsi qu'une réduction de l'hyperinsulinémie. De plus, les composés de l'invention réversent l'insulino-résistance induite par l'amyline.

Ils diminuent également l'hypertriglycéridémie et s'opposent à l'oxydation des LDL (Lipoprotéines de faible densité) d'où une implication dans la prévention des macroangiopathies.

Ils provoquent une réduction pondérale modeste, associée à une réduction de prise alimentaire.

Ces propriétés permettent de les utiliser en thérapeutique notamment pour le traitement des diabétiques non insulino dépendants non traités par un régime, des diabétiques non insulino dépendants traités par les médicaments hypoglycémiants, des diabétiques, insulino dépendants ou non, traités à l'insuline, ou des sujets non hyperglycémiques ayant une hyperinsulinémie (i.e. une obésité androïde) hypertendus ou non et présentant tous une résistance à l'insuline, induite ou non par l'amyline.

Ainsi les produits de l'invention sont utilisés dans le traitement du syndrome X (par le biais de l'amélioration de l'effet de l'insuline à la périphérie et/ou au niveau du foie, de la diminution des triglycérides et de l'oxydation des LDL, associé à une réduction pondérale modérée), et dans le traitement de l'hypertension chez les sujets insulinorésistants ou ayant des anomalies métaboliques telles que, par exemple, hyperinsulinémie, dyslipémie, hyperglycémie, associées ou non, secondaire ou non aux effets de l'amyline.

La présente invention a également pour objet les compositions pharmaceutiques contenant, comme principe actif, un dérivé de formule générale I ou un de ses sels physiologiquement tolérable, mélangé ou associé à un ou plusieurs excipients pharmaceutiques appropriés.

Les compositions pharmaceutiques ainsi obtenues se présentent généralement sous forme dosée contenant de 25 à 50 mg de principe actif. Elles peuvent revêtir la forme de comprimés, dragées, gélules, suppositoires, solutions injectables ou buvables et être administrées par la voie orale, rectale ou parentérale.

La posologie peut varier notablement selon l'âge et le poids du patient, la voie d'administration, la nature de la maladie et les traitements associés et s'échelonne de 25 à 50 mg par prise, 1 à 4 fois par jour.

Les exemples suivants illustrent l'invention.

## Exemple 1

Synthèse de (S)-1-(m-trifluorométhylphényl) 2-{β-{4-[2-(N-3,3-diphényl propionyl amino) éthyl] benzoyloxy} éthyl amino} propane, et son chlorhydrate.

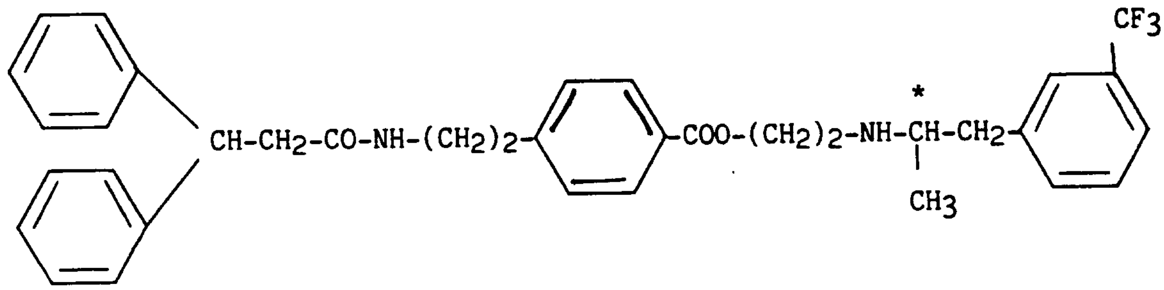

A 2,6 g d'hydrure de sodium en suspension à 50 % dans la paraffine et 200 ml de diméthylformamide anhydre, sont ajoutés, sous agitation en 1 heure, 18,7 g d'acide p-[2-(N-3,3-diphénylpropionyl amino) éthyl] benzoïque en solution dans 280 ml de diméthylformamide, puis on chauffe à 40 °C pendant 30 minutes. 17,8 g de (S)1-(m-trifluorométhylphényl) 2-(N-benzyl N-β-chloroéthyl amino) propane dans 500 ml de diméthylformamide sont ensuite ajoutés et le mélange réactionnel est chauffé à 100 °C pendant 24 heures. Après refroidissement, le solvant est évaporé sous vide. Le résidu est repris par 3 fois 100 ml de chlorure de méthylène. Après filtration et concentration, on obtient 29,3 g de (S)1-(m-trifluorométhylphényl) 2-{N-benzyl N-[β-{4-[2-(N-3,3-diphénylpropionyl amino) éthyl] benzoyloxy} éthyl] amino} propane.
Le produit ainsi obtenu est repris par 220 ml d'acide acétique et la solution est hydrogénée dans un appareil de PARR, sous $413.10^3$ Pa, en présence de 5 g de noir de carbone palladié à 5 % de Palladium, en chauffant à 50-60 °C pendant 2 heures. Après essorage, le solvant est éliminé sous vide, le résidu repris par 200 ml d'eau, et la solution neutralisée par 200 ml d'une solution à 10 % de $NaHCO_3$, en présence de 200 ml d'éther. La solution organique est lavée par 3 fois 50 ml d'eau, séchée sur sulfate de magnésium et concentrée sous vide. On obtient 23,5 g de produit qui chromatographié sur silice en éluant par $CH_2Cl_2/CH_3COOH$, 50/50 permet d'obtenir 11,5 g de (S)-1-(m-trifluorométhylphényl) 2-{β-4-[2-(N-3,3-diphénylpropionyl amino) éthyl] benzoyloxy} éthyl amino} propane.
A cette base en solution dans 600 ml d'éther anhydre, on ajoute 4,4 ml d'éther chlorhydrique 4,16 N dans 200 ml d'hexane. Le précipité formé est essoré, lavé à l'éther de pétrole et séché. On obtient 10,6 g de chlorhydrate de (S)-1-(m-trifluorométhylphényl)-2-{β-{4-[2-(N-3,3-diphényl propionyl amino) éthyl] benzoyloxy} éthyl amino} propane, P.F : 90 °C.
Le (S)1-(m-trifulorométhylphényl)-2-[N-benzyl N-(β-chloroéthyl) amino] propane de départ a été préparé comme suit :

a) 175,7 g (1 mole) de (S)N-(1-m-trifluorométhylphényl prop-2-yl) N-benzyl glycine dissous dans 500 ml de tétrahydrofurane sont ajoutés, en 3 heures, sous agitation, à 24 g d'hydrure de lithium aluminium en suspension dans 500 ml de tétrahydrofurane. La température se maintient à 40-42 °C. Après deux heures de chauffage à 40-45 °C, le mélange réactionnel est hydrolysé par 25 ml d'eau, 25 ml de NaOH 4N, puis par 75 ml d'eau. Après essorage et lavage avec du tétrahydrofurane, le filtrat est concentré à sec sous la pompe à vide. On obtient ainsi 166,5 g de (S)1-(m-trifluorométhylphényl) 2-[N-benzyl N-(β-hydroxyéthyl) amino] propane.
Au produit ainsi obtenu dissous dans 400 ml d'acétate d'éthyle anhydre et 150 ml de cyclohexane, on ajoute 113 ml d'éther chlorhydrique 4,6 N. Après essorage, le produit est lavé par un mélange acétate d'éthyle/cyclohexane (80/30), puis à l'éther de pétrole et séché à l'air. On obtient ainsi 144,5 g de chlorhydrate de (S)1-(m-trifluorométhylphényl) 2-[N-benzyl N-(β-hydroxyéthyl) amino] propane, P.F : 129-130 °C.
b) 37,4 g de ce chlorhydrate dans 100 ml de chloroforme anhydre sont ajoutés, en une heure, sous agitation, à 8 ml de chlorure de thionyle et 25 ml de chloroforme anhydre. Après un lent chauffage jusqu'au reflux, qui est atteint au bout de 1 heure 30 minutes, le reflux est maintenu pendant 1 heure. Après refroidissement et concentration sous vide, le résidu est recristallisé dans 220 ml d'acétate d'éthyle bouillant et dilué par 280 ml de cyclohexane. On obtient 20 g de chlorhydrate de (S)1-(m-trifluorométhylphényl) 2-[N-benzyl N-(β-chloroéthyl) amino] propane, P.F : 130-132 °C. 19,7 g de ce chlorhydrate, dissous dans 400 ml d'eau, sont alkalinisés par 7,5 ml d'une lessive de soude 36 ° Baumé, en présence de 400 ml d'éther. La couche organique est décantée et concentrée sous vide. On

obtient 17,9 g de (S)1-(m-trifluorométhyl phényl) 2-[N-benzyl N-(β-chloroéthyl) amino] propane, sous forme de base.

**Exemple 2**

**ETUDE PHARMACOLOGIQUE**

**A. ETUDE DE L'EFFET D'UN TRAITEMENT CHRONIQUE ADMINISTRÉ PER OS AU RAT SDCD MÂLE AGÉ DE 52 SEMAINES :**

**1. But de l'expérimentation**

Les essais sont réalisés sur des rats qui présentent des anomalies pondérales associées à un hyperinsulisme et une hyperglycéridémie.

Il est recherché :

- d'une part l'effet d'un traitement prolongé du produit de l'exemple 1 et d'une substance de référence sur ces anomalies, et
- d'autre part, la consommation du glucose par le tissu adipeux est mesuré à l'état basal et en présence d'insuline ($10^{-9}$ M).

**2. Protocole**

**2.1 Animaux utilisés**

Dans cette expérimentation, on a utilisé des rats mâles adultes SPRAGUE DAWLEY par lots de 5 à 12 rats.

Les rats utilisés, agés de 52 semaines, présentent

- une diminution de la tolérance au glucose,
- une augmentation de l'insulinémie basale, et
- une augmentation des lipides plasmatiques.

La stabulation (de 9 à 52 semaines) de ces rats a été réalisée dans une pièce à température régulée de 21 à 22 °C et soumise à un cycle fixe de lumière ( de 7 h 30 à 19 h 30) et d'obscurité (de 19 h 30 à 7 h 30). Leur alimentation a consisté en un régime d'entretien (UAR A 03) ; eau et nourriture ont été fournies "ad libitum", à l'exception du jeûne nocturne précédant les tests où la nourriture était retirée.

**2.2 Méthodes**

9 jours avant le début de l'expérimentation ($J_{-9}$), sont constitués des lots de rats par randomisation stratifiée sur le poids.

5 jours avant le début de l'expérimentation ($J_{-5}$), les rats sont conditionnés par administration d'une solution de gomme.

Le premier jour de l'expérimentation ($J_1$), les produits à tester sont administrés à différentes doses aux rats 2 fois par jour. Plus précisémment, les produits sont administrés en suspension dans la gomme entre 9 et 10 heures et 16-17 heures pendant 14 jours. Une pesée quotidienne des animaux traités est effectuée.

Le 15ème jour, les rats (à jeun depuis 18 heures) sont sacrifiés par décapitation. Le sang est recueilli directement dans une cupule. Un aliquote (50 µl) est transféré dans 500 µl d'acétate d'uranyl pour dosage de la glycémie. Un aliquote de 3 ml est transféré dans un tube contenant une solution d'héparine (30 µl pour 1 ml de sang total) et centrifugé pour séparer le plasma. Un autre aliquote de 300 µl est transféré dans un tube contenant 15 µl d'une solution d'EDTA/NaF pour dosage des lactates.

Le tissu adipeux épididymaire est prélevé pour étude métabolique immédiatement après sacrifice.

Pour chaque animal, deux fragments de tissu épididymaire droit et gauche sont émincés aux ciseaux et répartis dans 6 fioles d'incubation. Trois de ces fioles contenant 500 µl de milieu et les autres 500 µl de milieu additionné d'insuline de porc ($10^{-9}$ M), la production de $CO_2$ est donc mesurée en triplicate pour chacun des rats de chaque groupe.

### 2.3 Résultats

Ils sont répertoriés dans le tableau I ci-après.

Tableau I

| Traitement Rats SDCD mâles de 52 semaines | Variation pondérale % | Insulinémie basale µU/ml variation % | Glycémie g/l variation % | Tolérance au glucose K $10^{-2}$ variation % | Triglycéride g/l variation % | Cholestérol g/l variation % |
|---|---|---|---|---|---|---|
| **Contrôle** | + 0,4 % | 32 ± 2,4 | 1,06 ± 0,05 | 3,01 ± 0,24 | 3,64 ± 0,57 | 1,1 ± 0,14 |
| **Benfluorex** | | | | | | |
| 1 mg/kg×2 | - 2 % (NS) | - 10% NS | - 10 % NS | + 30 % p<0,05 | - 43 % p=0,025 | - 20 % NS |
| 2,5 mg/kg×2 | - 6 % p=0,85 | - 16% NS | - 12 % NS | + 25 % p=0,05 | - 44 % p=0,05 | - 30 % NS |
| **Produit de l'exemple 1** | | | | | | |
| 1,0 mg/kg×2 | - 1,4 % p=0,01 | - 15 % NS | + 8 % NS | + 23 % p p<0,05 | - 10 % NS | 0 % |
| 2,5 mg/kg×2 | - 3 % p=0,05 | - 26 % p=0,09 | 0 % | + 30 % p<0,05 | - 20 % p=0,09 | 0 % |

## B- ETUDE DE L'OXYDATION DES LDL IN VITRO

Une étude comparative entre le produit de l'exemple 1 et les substances de référence (Benfluorex, Diméthylbiguanide) sur l'oxydation des LDL in vitro a été réalisée.

Les résultats sont répertoriés dans le tableau II ci-après.

Tableau II

| | Oxydation des LDL in vitro | | Incorporation d'oléate dans les esters de cholestérol |
|---|---|---|---|
| | par le cuivre | par les monocytes | |
| Contrôle | | | |
| Benfluorex | Inactif à | Inactif à | - 30 % à $10^{-5}$ M |
| Diméthylbiguanide $10^{-4}$ M | Inactif à $10^{-4}$ M | Inactif à $10^{-4}$ M | Inactif à $10^{-5}$ M |
| Produit de l'exemple 1 | $IC_{50} = 5.10^{-5}$ M | $IC_{50} = 5.10^{-5}$ M | - 70 % à $10^{-5}$ M |

## C. RECHERCHE D'UN EFFET HYPOTENSEUR CHEZ LE CHIEN ÉVEILLÉ

La pression artérielle a été mesurée par brassard externe (Sphygmotensiomètre) au niveau de la queue du chien avant et après traitement avec le produit de l'exemple 1, à la dose de 5 mg/kg P.O.

Les résultats sont répertoriés dans le tableau III ci-après.

Tableau III

| Produit testé (dose) | Animal traité (nombre : n) | Diminution de la pression artérielle | |
|---|---|---|---|
| | | PA systolique | PA diastolique |
| Produit de l'exemple 1 (5 mg/kg P.O) | chien (n = 4) | -15 à -22 (mmHg) | -20 à -27 (mmHg) |

## D. CONCLUSION

Les résultats des études ci-dessus décrites montrent l'intérêt pharmacologique et thérapeutique de l'isomère dextrogyre (ou énantiomère S) du chlorhydrate de 1-(m-trifluorométhylphényl) 2-{β-{4-[2-(N-3,3-diphényl-propionyl amino) éthyl] benzoyloxy} éthylamino} propane (dit produit de l'exemple 1) et l'originalité et la supériorité de ce dernier vis à vis de substances de référence reconnues comme spécifiquement adaptées aux traitements concernés.

## Revendications

**Revendications pour les Etats contractants suivants : AT, BE, CH, DE, DK, FR, GB, GR, IT, LI, LU, NL, PT, SE, IE**

**1.** Les dérivés de benzoate d'éthanolamine de formule générale I :

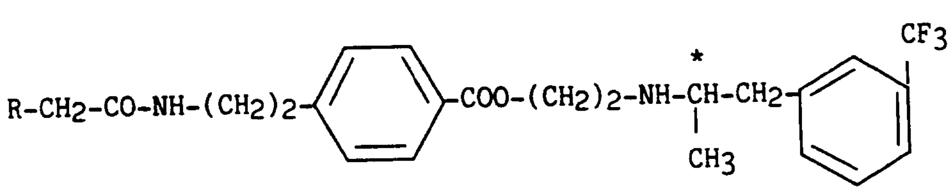

(I)

dans laquelle R représente :

a) soit un radical de formule :

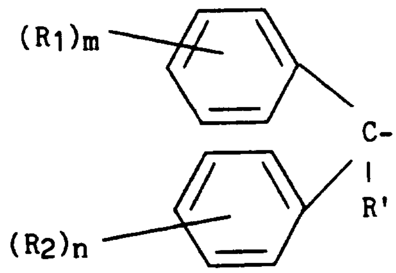

dans laquelle :

- R' represente un atome d'hydrogène ou un radical alkyle contenant de 1 à 5 atomes de carbone en chaine droite ou ramifiée,
- $R_1$ et $R_2$, identiques ou différents, représentent chacun un atome d'hydrogène ou un radical alkyle ou alkoxy ayant chacun de 1 à 5 atomes de carbone en chaine droite ou ramifiée, et
- m et n, identiques ou différents, représentent chacun 1, 2 ou 3 ;

b) soit un radical fluorényle de formule :

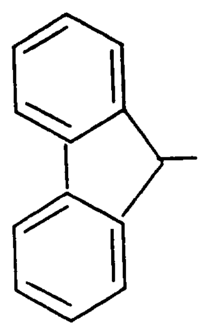

sous forme de composé racémique ou d'énantiomères.

**2.** Les sels physiologiquement tolérables des dérivés de la revendication 1 avec des acides appropriés.

**3.** Le (S)-1-(m-trifluorométhylphényl) 2-{β-{4-[2-(N-3,3-diphénylpropionyl amino) éthyl] benzoyloxy} éthyl amino} propane, et son chlorhydrate.

**4.** Le procédé de préparation des dérivés de la revendication 1 caractérisé en ce que :

- l'on transforme l'acide de formule générale II :

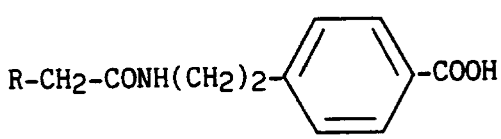

(II)

dans laquelle R a la signification définie dans la revendication 1, en un sel de formule générale II' :

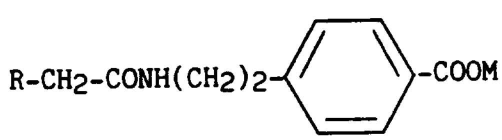

(II')

dans laquelle R a la signification définie dans la revendication 1 et M est un métal alcalin ou alcalinoterreux ;

- l'on fait réagir ce dernier avec un composé halogéné de formule générale III :

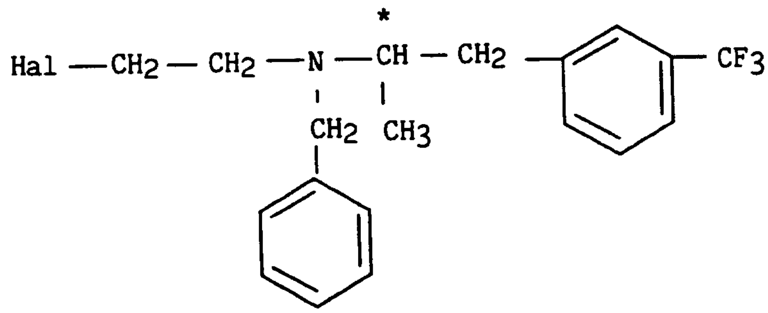

(III)

dans laquelle Hal représente un atome d'halogène ;

- et l'on débenzyle catalytiquement le composé ainsi obtenu de formule générale IV :

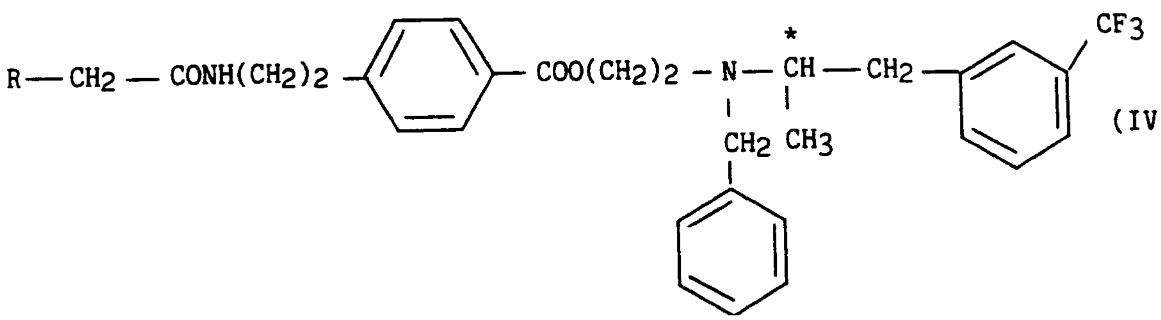

(IV)

dans laquelle R a la signification définie dans la revendication 1.

**5.** Les compositions pharmaceutiques contenant comme principe actif un dérivé selon les revendications 1 à 3 avec un ou plusieurs excipients pharmaceutiques appropriés.

**6.** Les compositions pharmaceutiques selon la revendication 5 présentées sous une forme convenant notamment dans le traitement du syndrome X, secondaire ou non à une hyperamylinémie associant une diminution de la tolérance au glucose, hyperinsulinémie, dyslipémie et hypertension et dans le traitement de l'hypertension chez les sujets insulinorésistants ou ayant une ou plusieurs anomalies métaboliques.

**Revendication pour l'Etat contractant suivant : ES**

**1.** Le procédé de préparation des dérivés de benzoate d'éthanolamine de formule générale I :

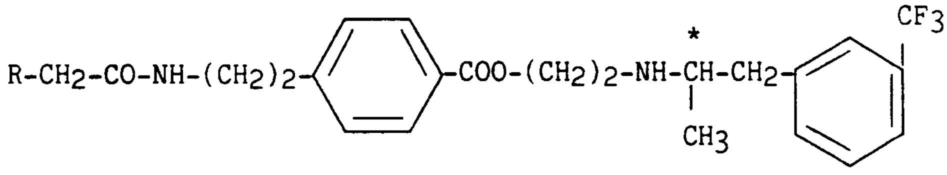

(I)

dans laquelle R représente :

a) soit un radical de formule :

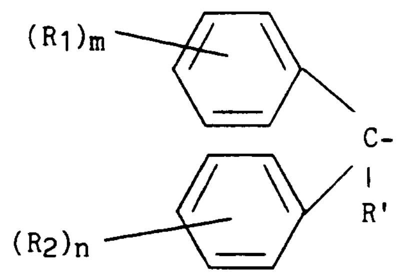

dans laquelle :

- R' represente un atome d'hydrogène ou un radical alkyle contenant de 1 à 5 atomes de carbone en chaine droite ou ramifiée,
- $R_1$ et $R_2$, identiques ou différents, représentent chacun un atome d'hydrogène ou un radical alkyle ou alkoxy ayant chacun de 1 à 5 atomes de carbone en chaine droite ou ramifiée, et
- m et n, identiques ou différents, représentent chacun 1, 2 ou 3 ;

b) soit un radical fluorényle de formule :

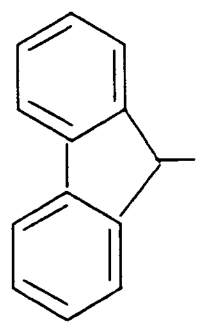

sous forme de composé racémique ou d'énantiomères, et leurs sels physiologiquement tolérables avec des acides appropriés, caractérisé en ce que :

- l'on transforme l'acide de formule générale II :

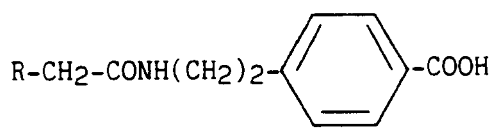

(II)

dans laquelle R a la signification précédemment définie en un sel de formule générale II' :

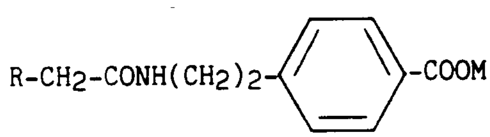

(II')

dans laquelle R a la signification précédemment définie et M est un métal alcalin ou alcalino-terreux ;

- l'on fait réagir ce dernier avec un composé halogéné de formule générale III :

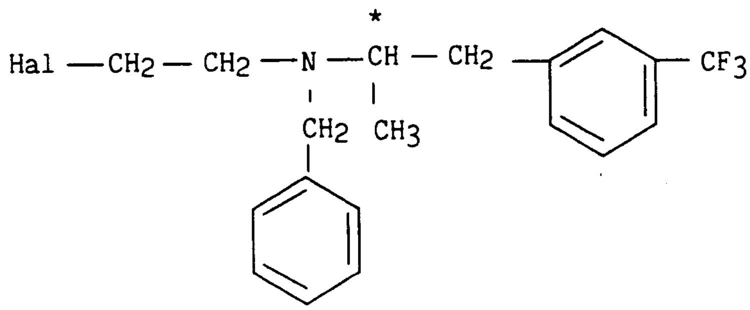

(III)

dans laquelle Hal représente un atome d'halogène ;

- et l'on débenzyle catalytiquement le composé ainsi obtenu de formule générale IV :

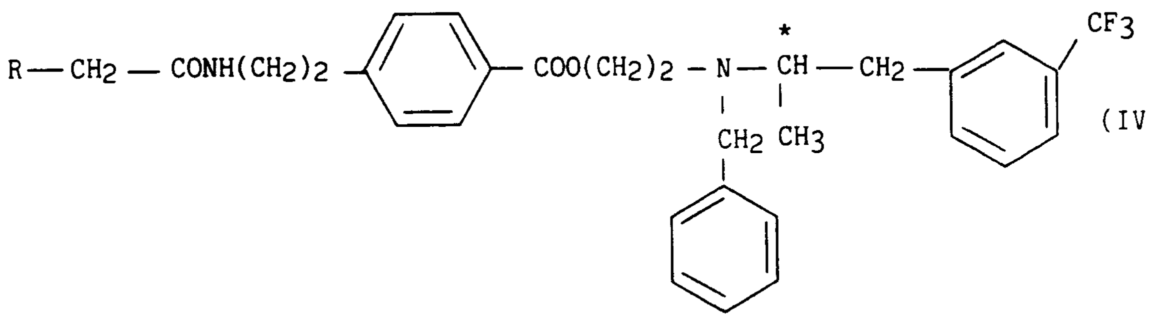

(IV)

dans laquelle R a la signification précédemment définie,

et, si on le désire, on traite les dérivés ainsi obtenus avec des acides physiologiquement tolérables pour obtenir les sels d'addition acides correspondants.

**Claims**

**Claims for the following Contracting States : AT, BE, CH, DE, DK, FR, GB, GR, IT, LI, LU, NL, PT, SE, IE**

**1.** Ethanolamine benzoate compounds of the general formula I :

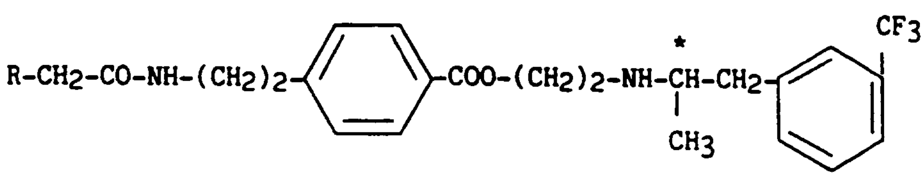

(I)

wherein R represents :

a) either a radical of the formula :

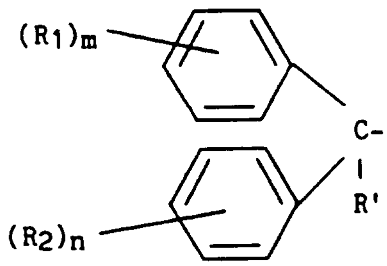

wherein :

- R' represents a hydrogen atom or a straight-chain or branched alkyl radical containing from 1 to 5 carbon atoms,
- $R_1$ and $R_2$, which are the same or different, each represents a hydrogen atom or a straight-chain or branched alkyl or alkoxy radical each having from 1 to 5 carbon atoms, and
  m and n, which are the same or different, each represents 1, 2 or 3;

b) or a fluorenyl radical of the formula :

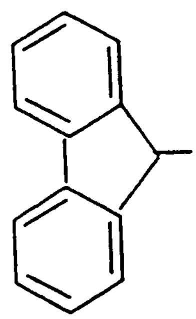

in the form of a racemic compound or of enantiomers.

**2.** The physiologically tolerable salts of compounds of claim 1 with appropriate acids.

**3.** S-1-(m-trifluoromethylphenyl)-2-{β-{4-[2-(N-(3,3-diphenylpropionyl)amino)ethyl]-benzoyloxy}ethylamino}propane and its hydrochloride.

**4.** A process for the preparation of the compounds of claim 1, characterised in that :

- the acid of the general formula II :

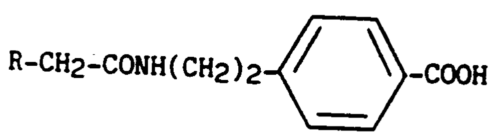

(II)

wherein R is as defined in claim 1, is converted into a salt of the general formula II' :

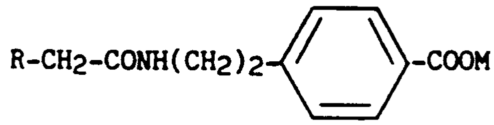

(II')

wherein R is as defined in claim 1 and M represents an alkali metal or alkaline earth metal;

- the latter is reacted with a halogenated compound of the general formula III :

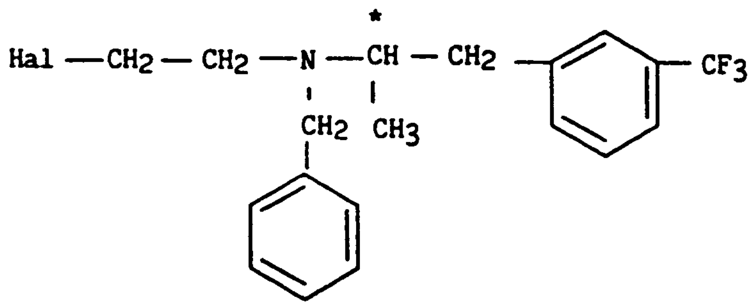

(III)

wherein Hal represents a halogen atom;

- and the compound so obtained of the general formula IV:

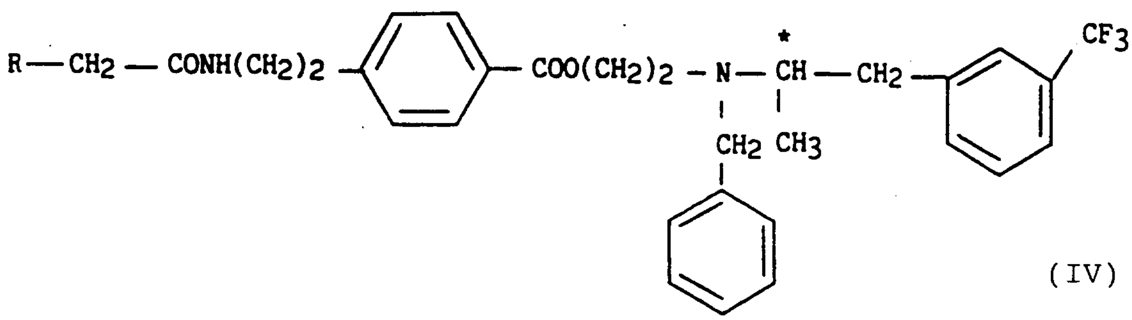

(IV)

wherein R is as defined in claim 1, is catalytically debenzylated.

5. Pharmaceutical compositions containing as active ingredient a compound according to any one of claims 1 to 3 together with one or more appropriate pharmaceutical excipients.

6. The pharmaceutical compositions according to claim 5 presented in a form suitable especially for the treatment of syndrome X, secondary or not to a hyperamylasaemia with which there is associated a decrease in glucose tolerance, hyperinsulinaemia, dyslipaemia and hypertension, and for the treatment of hypertension in patients who are insulin-resistant or have one or more metabolic anomalies.

**Claim for the following Contracting State : ES**

1. A process for the preparation of ethanolamine benzoate compounds of the general formula I:

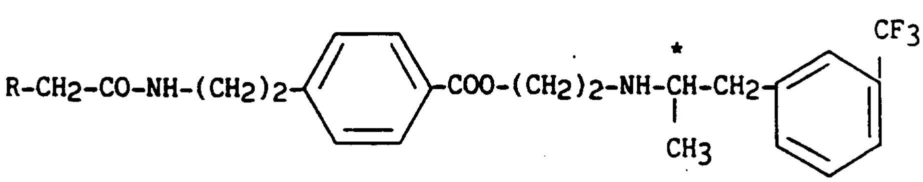

(I)

wherein R represents :

a) either a radical of the formula :

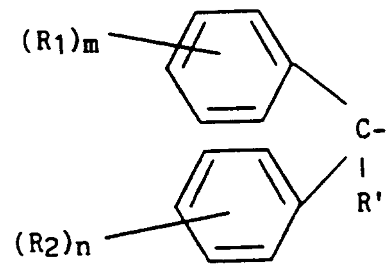

wherein :

- R' represents a hydrogen atom or a straight-chain or branched alkyl radical containing from 1 to 5 carbon atoms,
- $R_1$ and $R_2$, which are the same or different, each represents a hydrogen atom or a straight-chain or branched alkyl or alkoxy radical each having from 1 to 5 carbon atoms, and

  m and n, which are the same or different, each represents 1, 2 or 3;

b) or a fluorenyl radical of the formula :

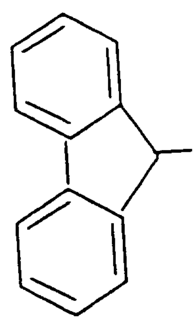

in the form of a racemic compound or of enantiomers, and their physiologically tolerable salts with appropriate acids, characterised in that :

- the acid of the general formula II :

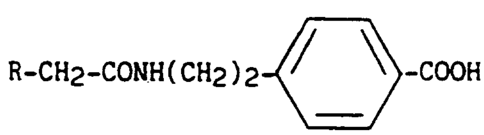

(II)

wherein R is as defined above, is converted into a salt of the general formula II' :

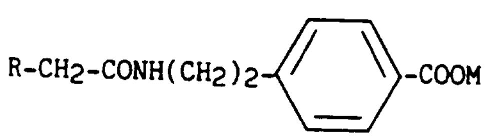

(II')

wherein R is as defined above and M represents an alkali metal or alkaline earth metal;

- the latter is reacted with a halogenated compound of the general formula III :

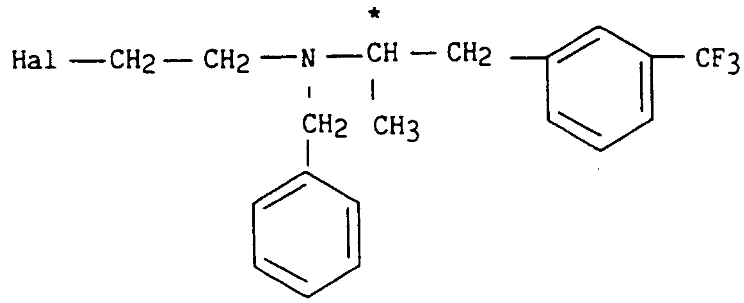

(III)

wherein Hal represents a halogen atom;

- and the compound so obtained of the general formula IV:

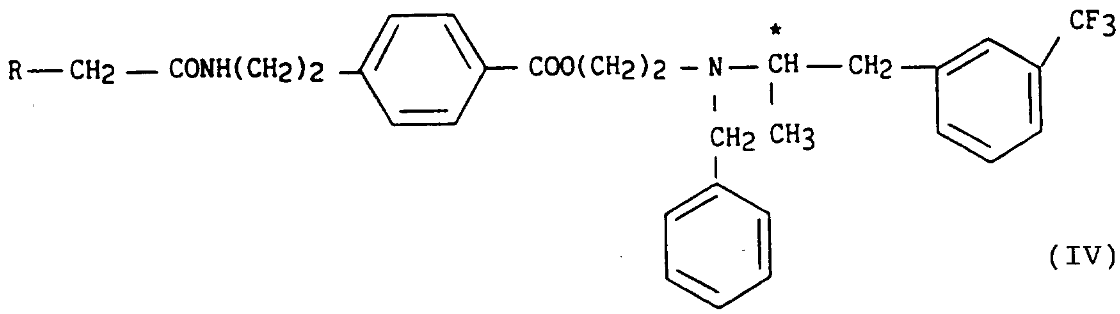

(IV)

wherein R is as defined above, is catalytically debenzylated,

and, if desired, the compounds so obtained are treated with physiologically tolerable acids to obtain the corresponding acid addition salts.

**Patentansprüche**

**Patentansprüche für folgende Vertragsstaaten : AT, BE, CH, DE, DK, FR, GB, GR, IT, LI, LU, NL, PT, SE, IE**

**1.** Ethanolamin-benzoat-Derivate der allgemeinen Formel I:

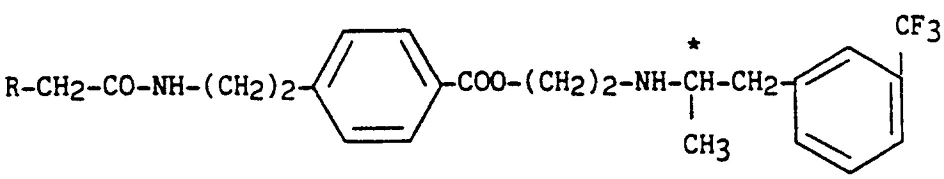

(I)

in der R:

a) entweder eine Gruppe der Formel:

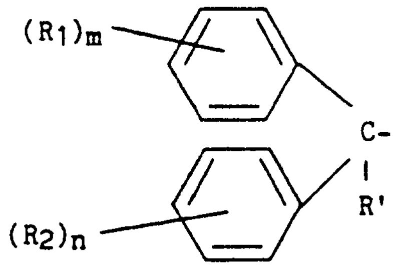

in der:

- R' ein Wasserstoffatom oder eine Alkylgruppe mit 1 bis 5 Kohlenstoffatomen in gerader oder verzwelgter Kette,
- $R_1$ und $R_2$, die gleichartig oder verschieden sein können, jeweils ein Wasserstoffatom oder eine Alkyl- oder Alkoxygruppe mit 1 bis 5 Kohlenstoffatomen in gerader oder verzweigter Kette und
- m und n, die gleichartig oderverschieden sein können, jeweils 1, 2 oder 3 darstellen;

b) oder eine Fluorenylgruppe der Formel:

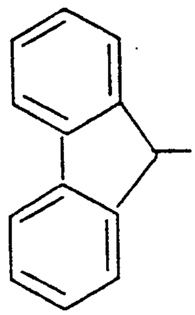

bedeutet,

in Form der racemischen Verbindung oder der Enantiomeren.

**2.** Die Physiologisch verträglichen Salze der Derivate nach Anspruch 1 mit geeigneten Säuren.

**3.** (S)-1-(m-Trifluormethylphenyl)-2-{$\beta$-{4-[2-(N-3,3-diphenylpropionylamino)-ethyl]-benzoyloxy}-ethylamino}-propan und dessen Hydrochlorid.

**4.** Verfahren zur Herstellung der Derivate nach Anspruch 1, **dadurch gekennzeichnet**, daß man

- die Säure der allgemeinen Formel II:

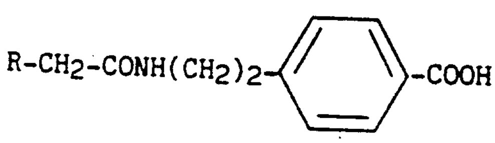

(II)

in der R die in Anspruch 1 angegebenen Bedeutungen besitzt, In ein Salz der allgemeinen Formel II':

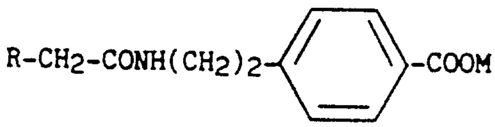

(II')

in der R die in Anspruch 1 angegebenen Bedeutungen besitzt und M ein Alkalimetall oder Edalkalimetall darstellt, umwandelt;

- dieses letztere mit einer Halogenverbindung der allgemeinen Formel III:

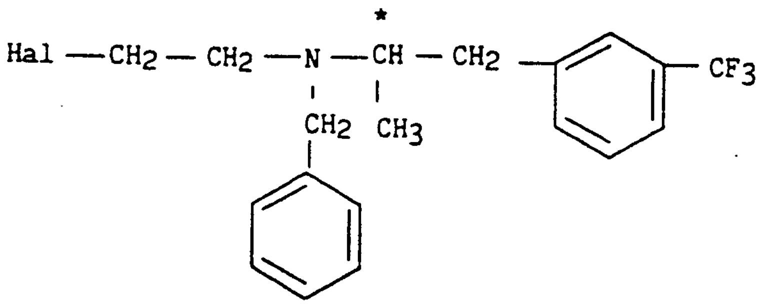

(III)

worin Hal ein Halogenatom darstellt, umsetzt;

- und die in dieser Weise erhaltene Verbindung der allgemeinen Formel IV:

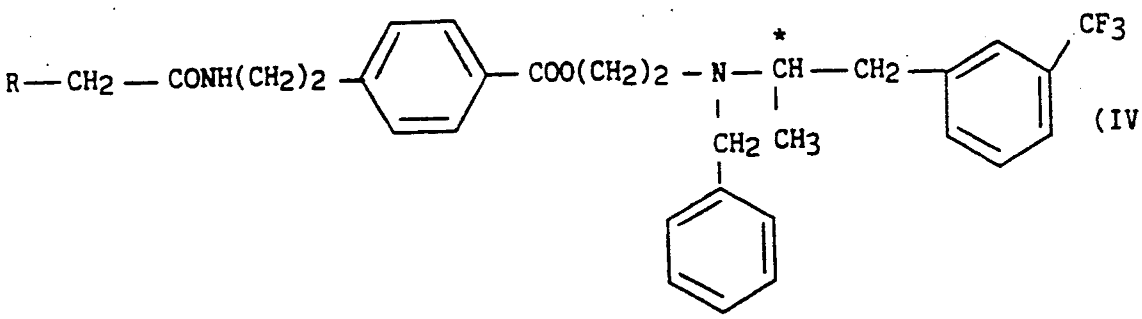

(IV)

in der R die in Anspruch 1 angegebenen Bedeutungen besitzt, katalytisch debenzyliert.

5. Pharmazeutische Zubereitungen enthaltend als Wirkstoff ein Derivat nach einem der Ansprüche 1 bis 3 mit einem oder mehreren geeigneten pharmazeutischen Trägermateriallen.

6. Pharmazeutische Zubereitungen nach Anspruch 5 in einer Form, die insbesondere bei der Behandlung des bezuglich einer Hyperamylinämie gegebenenfalls sekundären Syndroms X, welche zu einer Verminderung der Glucoseverträglichkeit führt, der Hyperinsulinämie, der Dyslipidämie und der Hypertension und bei der Behandlung der Hypertension bei insulinresistenten Patienten oder solche, die eine oder mehrere Stoffwechselanomalien aufweisen, geeignet ist.

**Patentanspruch für folgenden Vertragsstaat : ES**

1. Verfahren zur Herstellung der Ethanolamin-benzoat-Derivate der allgemeinen Formel I:

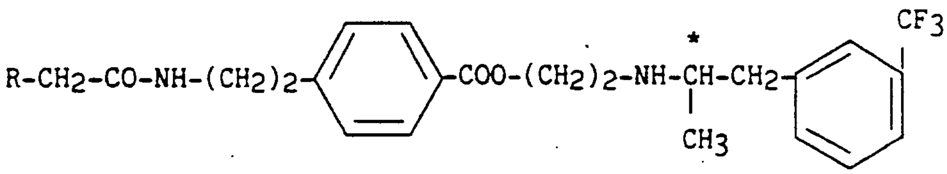

(I)

in der R:

a) entweder eine Gruppe der Formel:

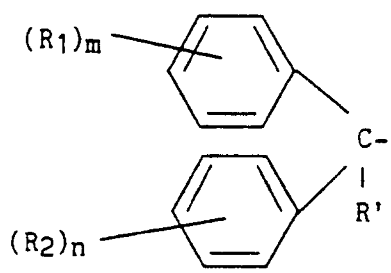

in der:

- R' ein Wasserstoffatom oder eine Alkylgruppe mit 1 bis 5 Kohlenstoffatomen in gerader oder verzweigter Kette,
- $R_1$ und $R_2$, die gleichartig oder verschieden sein können, jeweils ein Wasserstoffatom oder eine Alkyl- oder Alkoxygruppe mit 1 bis 5 Kohlenstoffatomen in gerader oder verzweigter Kette und
- m und n, die gleichartig oder verschieden sein können, jeweils 1, 2 oder 3 darstellen;

b) oder eine Fluorenylgruppe der Formel:

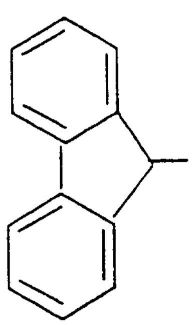

bedeutet,

in Form der racemischen Verbindung oder der Enantiomeren und von deren physiologisch verträglichen Salzen mit geeigneten Säuren, **dadurch gekennzeichnet,** daß man

- die Säure der allgemeinen Formel II:

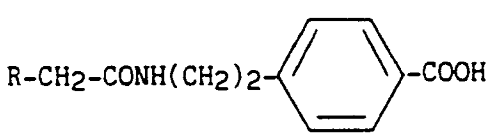

(II)

in der R die oben angegebenen Bedeutungen besitzt, in ein Salz der allgemeinen Formel II':

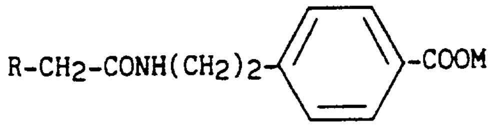

(II')

In der R die oben angegebenen Bedeutungen besitzt und M ein Alkalimetall oder Edalkalimetall darstellt, umwandelt;

- dieses letztere mit einer Halogenverbindung der allgemeinen Formel III:

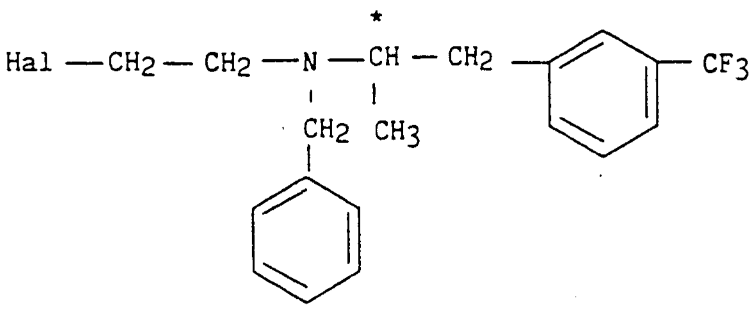

(III)

worin Hal ein Halogenatom darstellt, umsetzt;

- und die in dieser Weise erhaltene Verbindung der allgemeinen Formel IV:

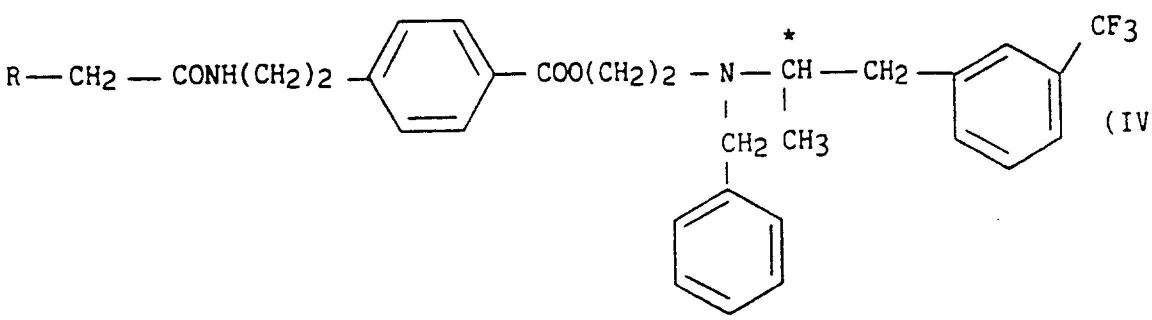

(IV)

in der R die oben angegebenen Bedeutungen besitzt, katalytisch debenzyliert und gewünschtenfalls die in dieser Weise erhaltenen Derivate mit physiologisch verträglichen Säuren behandelt zur Bildung der entsprechenden Säureadditionssalze.